# EUROPEAN PATENT SPECIFICATION

(11) **EP 3 253 365 B1**
(45) Date of publication and mention of the grant of the patent: **18.02.2026**
(21) Application number: 16702437.1
(22) Date of filing: 02.02.2016
(51) Int. Cl.: A61K 9/00, A61P 27/00, A61K 9/107, A61K 31/335, A61K 31/55, A61K 31/573, A61K 31/7048, A61K 38/13, A61K 45/06, A61P 27/02

(54) **POLYAPHRONS AND PALPEBRAL ADMINISTRATION THEREOF**
POLYAPHRONE UND DEREN PALPEBRALERVERABREICHUNG
POLYAPHRONES ET LEUR ADMINISTRATION PALPEBRALE

(30) Priority: 02.02.2015 US 201562110740 P
(43) Date of publication of application: 13.12.2017
(73) Proprietor: Santen Pharmaceutical Co., Ltd., Osaka-shi, Osaka 530-8552 (JP); Santen SAS, 92800 Puteaux (FR)
(72) Inventor: SCHMITT, Mathieu, 75017 Paris (FR); KIDO, Kazutaka, Osaka-shi Osaka 530-8552 (JP); INAGAKI, Koji, Ikoma-shi Nara 630-0101 (JP); BOUTTAZ, Adeline, 91190 Gif-sur-Yvette (FR)
(74) Representative: Icosa
(86) International application number: PCT/EP2016/052188
(87) International publication number: WO 2016/124601

(56) References cited:
- EP-A1- 1 970 047
- US-A- 4 999 198
- US-A1- 2010 260 835
- US-A1- 2011 104 206
- US-A1- 2013 345 150
- US-B2- 8 603 503

## Description

### FIELD OF INVENTION

The present invention relates to easy-to-apply polyaphrons in the general domain of galenic formulation. Especially, the present invention relates to compositions containing at least one polyaphron for topical administration, namely palpebral administration.

### BACKGROUND OF THE INVENTION

### Presentation of polyaphrons

Polyaphrons are dispersions. In the meaning of the present invention, by "dispersion", it is meant a system in which liquid globules or solid particles are dispersed in a continuous phase. In the present invention, the term "polyaphron" refers to liquid globules called "aphrons" dispersed in a continuous phase wherein the aphrons are not miscible. The dispersed phase may be a hydrophilic phase or a hydrophobic phase, provided that the aphrons remains non miscible in the continuous phase. The continuous phase may be fluid, liquid or gel.

More precisely, the dispersed phase of polyaphrons may be composed of complex aphrons dispersed in a continuous phase, said aphrons having:
- a core made composed of a material non-miscible with the continuous phase;
- an intermediate layer composed of the same material as the continuous phase;
- an outer layer including surfactants.

For example, in an oil-in-water polyaphron, aphrons may include an inner core, which is an hydrophobic globule, an intermediate layer made of an aqueous solution and an outer layer including surfactants. In an oil-in-water polyaphron, the hydrophobic phase may represent up to 98% in weight by total weight of polyaphron, and the polyaphron includes a very low amount of surfactants considering the large amount of dispersed phase (typically ranging from 0.05 to 5%, or 0.1 to 3% in weight by total weight of polyaphron).

As another example, in a water-in-oil polyaphron, aphrons may include an inner core, which is an aqueous globule, an intermediate layer made of a hydrophobic solution and an outer layer including surfactants. In a water-in-oil polyaphron, the hydrophilic phase can represent up to 98% in weight by total weight of polyaphron.

It is worth noting that, contrarily to emulsions characterized by a single interface, polyaphrons may have a multi-layer structural architecture which may be responsible for their remarkable stability.

### Background

Polyaphrons were first described about 40 years ago. US 4,486,333 discloses a method of preparation of a polyaphron, especially oil-in-water polyaphrons using kerosene, petroleum ether, carbon tetrachloride, carbon tetrachloride-cyclohexane mixture as hydrophobic phase and water or methanol as hydrophilic phase.

Polyaphrons compositions are known as oral drug delivery systems. For example, WO 2005/011628 discloses the delivery of lipophilic poorly water-soluble drugs in immediate dosage form as polyaphron compositions.

Polyaphron compositions can be used as topical drug delivery systems for transporting active ingredients. For example, US 4,999,198 discloses the delivery of scopolamine dissolved in peanut and mineral oil to another medium; WO 2008/110826 carries corticosteroids combined with vitamin E through topical application by application of the skin; EP 1 970 049 discloses topical composition for dermal use of composition including vitamin D in treatment of a number of skin conditions such as psoriasis or dermatitis.

Recently, polyaphron dispersions have been used for ophthalmic purposes. For example, WO 2012/123515 discloses a method for delivering different active ingredients such as antibiotics (cyclosporine, vancomycin), anti-inflammatory compounds (flurbiprofen, fluticasone), prostaglandin (latanoprost). WO 2012/123515 discloses eye drops to be administered topically to the human/animal cornea.

US 2011/0104206 A1 describes topical compositions comprising a muscle fasciculating agent, such as for example gels, creams, ointment or lotions. US 2011/0104206 A1 describes that therapeutic agents may be released into the eyes or systemically by administering these compositions to the outer surface of the eyelid of a subject. US 2011/0104206 A1 does not disclose nor suggest that the compositions for eyelid administration may comprise polyaphrons, even less be formulated as polyaphrons.

### Technical issue

The Applicant observed that various problems were arising when eye drops were administered topically directly to the eye surface of the patient. First, some patients are not able to administer themselves eye drops. Problems of topical administration arise. Also, when dispensing eye drops, the patient wonders whether or not at least one eye drop has reached the target (the cornea and/or the conjunctiva); this problem increases with elderly and pediatric populations. As a consequence, problems of accurate dosing arise. Also, even when the drops are properly delivered to the surface of the eye, the patient may feel a certain discomfort or experience a blurred vision. Instilling eye drops on the ocular surface usually triggers a reflex blinking, washing out most of the eye drops within a few seconds. It is admitted that more than 95% of the eye drops is washed away from the ocular surface within 2 minutes following administration.

More importantly, the direct application of a composition to the surface of the eye may lead to irritation, especially corneal and/or conjunctival irritation due to the presence of irritating ingredients in the formulation. Such consequences may be a real issue when the principal target of the treatment is the eye.

Another critical issue in eye treatment is the frequency of administration. Applying eye drops several times a day may become a real burden to some patients, and patient compliancy to treatment may drastically lower.

These different issues relative to eye treating composition brought the Applicant to conceive and reduce to practice an easy-to-apply polyaphron able to target the eye while avoiding direct application of the composition and the cornea, and also avoiding discomfort, blurred vision and irritation.

Another issue in eye treatment is the achievement of an extended release for therapeutic agents onto the eye, in order to progressively deliver said therapeutic agent to the targeted part of the eye. The administration of a high quantity of an active agent onto the eye in a short duration can lead to toxic concentration and consequently be harmful for the targeted part of the eye.

Therefore, there is an interest in releasing a quantity of therapeutic agent over a sustained period, said quantity being sufficient to achieve the treatment efficacy, without releasing an excess of therapeutic agent which may cause local toxicity. Another advantage of an extended delivery duration is that the frequency of administration may be reduced and the compliance improved consequently.

Surprisingly, the Applicant realized that polyaphrons could be an efficient vehicle for sustained and/or controlled releasing of drug substances to the eye of a subject via palpebral application. This finding brought the Applicant to conceive and reduce to practice a polyaphron able to extendedly release a drug substance in a sustainable and/or controlled manner onto the eye of a subject.

### SUMMARY

The present invention relates to a composition for use in the treatment of an eye disease or an eye condition of a subject by palpebral administration of the composition onto the upper and/or lower eyelid of the subject, the composition comprising a polyaphron, as described in the claims.

The present invention further relates to a kit comprising a container containing a composition for use according to the present invention as described in the claims and a spreading device for the application of the composition. In one embodiment, the spreading device for the application of the composition is a brush or an applicator

### DETAILED DESCRIPTION

### Polyaphron

The invention thus relates to a polyaphron comprising at least one hydrophilic phase, at least one hydrophobic phase, and at least one surfactant. The polyaphron of the invention comprises:
- at least one hydrophilic phase;
- at least one hydrophobic phase;
- at least one surfactant selected from non-ionic surfactants;
- optionally at least one additive selected from antioxidants, osmotic, viscosity modulator agent, pH adjusting, buffering, preservative, solubilizers, chelating agents; and
- at least one active ingredient.

### Hydrophilic phase

According to one embodiment, the polyaphron of the invention is a polyaphron where the hydrophilic phase is an aqueous composition or water.

The aqueous composition may include water-miscible surfactants or polymers, and water.

### Hydrophobic phase

According to one embodiment, the polyaphron of the invention is an polyaphron where the hydrophobic phase comprises at least one of the group selected from short chain (C4 to C6) fatty acids mono- di- and triesters of glycerol, medium chain (C8 to C12) fatty acids mono-, di- and triesters of glycerol, long chain (C14 and more) saturated fatty acids mono-, di- and triesters of glycerol, long chain (C14 and more) unsaturated fatty acids mono-, di- and triesters of glycerol, vegetable oils, almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, shark liver oil, squalane, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, fatty acid esters (e.g. ethyl oleate, isopropyl myristate, isopropyl palmitate, isopropyl stearate, isopropyl isostearate...), short chain (C4 to C6) fatty acids mono- and diesters of propylene glycol, medium chain (C8 to C12) fatty acids mono- and diesters of propylene glycol, long chain (C14 and more) saturated fatty acids mono- and diesters of propylene glycol, long chain (C14 and more) unsaturated fatty acids mono- and diesters of propylene glycol, fatty alcohol (e.g. myristyl alcohol, oleyl alcohol...), branched fatty alcohol (e.g. octyldodecanol...), silicone oils, mineral oils, petrolatum, vitamin E, vitamin E acetate, tocopherol, tocopherol acetate, saturated fatty acids, unsaturated fatty acids, phospholipids.

Preferably, the hydrophobic phase of the polyaphron of the invention is or comprises a pharmaceutically acceptable oil or a pharmaceutically acceptable mixture of oils.

In one embodiment, hydrophobic phase of the polyaphron of the invention is free of phospholipids.

In one embodiment, the hydrophobic phase of the polyaphron of the invention comprises MCT. In one embodiment, the hydrophobic phase of the polyaphron of the invention consists of MCT.

In one embodiment, the hydrophobic phase of the polyaphron of the invention comprises mineral oils. In an embodiment, hydrophobic phase of the polyaphron of the invention consists of mineral oils.

In one embodiment, the hydrophobic phase of the polyaphron of the invention comprises triacetin.

### Surfactant

### Non-ionic surfactant

The polyaphron of the invention comprises at least one non-ionic surfactant. The at least one non-ionic surfactant is selected from the group of alkyl polyglycol ether, an alkyl polyglycol ester, an ethoxylated alcohol, a polyoxyethylene sorbitan fatty acid ester, castor oil derivatives, polyoxyethylene fatty acid ester, polyoxyethylene glycol hydrogenated castor oil, polyoxyethylene glycol castor oil, a sorbitan fatty acid ester (e.g. sorbitan monolaurate, sorbitan monooleate), a block copolymer of ethylene oxide and propylene oxide (e.g. poloxamer 188, poloxamer 407), poloxamers, tyloxapol, polysorbates, sucrose alkyl esters, sucrose alkyl ether, short chain (C4 to C6) fatty acids mono- and diesters of glycerol, medium chain (C8 to C12) fatty acids mono- and diesters of glycerol, long chain (C14 and more) saturated fatty acids mono- and diesters of glycerol, long chain (C14 and more) unsaturated fatty acids mono- and diesters of glycerol, short chain (C4 to C6) fatty acids monoesters of propylene glycol, medium chain (C8 to C12) fatty acids monoesters of propylene glycol, long chain (C14 and more) saturated fatty acids monoesters of propylene glycol, long chain (C14 and more) unsaturated fatty acids monoesters of propylene glycol, polyoxylglycerides, polyoxyethylene alkyl esters, polyoxyethylene ethers, vitamin E polyethylene glycol succinate, alkylpolyglycosides and mixtures thereof.

### Ionic surfactant

In one embodiment, the polyaphron comprises at least one ionic surfactant. The ionic surfactant may be a cationic surfactant or an anionic surfactant.

Advantageously, the at least one ionic surfactant is a cationic surfactant selected from the group of C10-C24 primary alkylamines, tertiary aliphatic amines, quaternary ammonium compounds, cationic lipids (e.g. phosphatidyl choline), amino alcohols, biguanide salts, cationic polymers and the mixture of two or more thereof. In a preferred embodiment, the at least one cationic agent is a quaternary ammonium compound preferably selected from the group consisting of benzalkonium halide, lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetalkonium halide, cetethyldimonium halide, cetylpyridinium halide, benzododecinium halide, chlorallyl methenamine halide, myristalkonium halide, stearalkonium halide or a mixture of two or more thereof, halide being preferably chloride or bromide.

In one embodiment, the at least one ionic surfactant is an anionic surfactant selected from the group of phospholipids, lecithins, perfluorooctanoate, perfluorooctanesulfonate, alkyl sulfate salts, sodium lauryl ether sulfate, alkyl benzene sulfonate, soaps or fatty acid salts or mixtures thereof.

### Amounts of surfactants

The polyaphron of the invention comprises at least one surfactant. Advantageously, the amount of surfactant in the polyaphron of the invention ranges from 0.005 to 5%, preferably from 0.05 to 5% in weight by total weight of polyaphron. Generally, in conventional emulsions, the ratio surfactant to oil ranges from 1/10 to 2/1. In the polyaphron of the invention, the ratio surfactant to oil in polyaphron ranges from 1/50 to 1/40; the amount of surfactant is therefore much lower in the polyaphron of the invention than in conventional emulsions. This difference gives the polyaphron of the invention an obvious advantage over emulsions in terms of limited surfactant-related side effects.

### Additives

In one embodiment, the polyaphron comprises additives selected from the group of antioxidants, osmotic, viscosity modulator agent, pH adjusting agent or buffer, preservative, solubilizers, chelating agents. The amount of additives may be calculated by the skilled artisan with respect to the Pharmacopeia and biological criteria.

### Antioxidants

In one embodiment, the polyaphron includes antioxidants selected from the group of vitamin E, sodium bisulfite, sodium metasulfite, sodium thiosulfate anhydrous, citric acid monohydrate, ascorbyl palmitate and ascorbic acid, butylhydroxyltoluene, butylhydroxyanisole, propylgallate. These antioxidants can be used solely or in combination.

The amount of antioxidant may be calculated by the skilled artisan with respect to the Pharmacopeia criteria and biological criteria.

### Osmotic agents

In one embodiment, the polyaphron includes at least one osmotic agent selected from the group of glycerol, propylene glycol, sodium chloride, potassium chloride, sorbitol, mannitol, xylitol and the like. Also, the amount of osmotic agent is determined in respect of the Pharmacopeia criteria and biological criteria.

### Viscosity modulator agent - viscosity of the polyaphron of the invention

In one embodiment, the polyaphron includes at least one viscosity modulator agent selected from the group of carbomers, polycarbophil, cellulose derivatives (e.g. hydroxypropylcellule, hydroxypropylmethylcellulose, carboxymethylcellulose...), povidone, copovidone, natural gums (e.g. gellan gum, guar gum, xanthan gum, agar agar, xyloglucan...), poloxamer and like. These viscosity modulator agents can be used solely or in combination, the amount fulfilling the requirement of the Pharmacopeia (in Europe and in the U.S.) and biological criteria.

In one embodiment, the polyaphron of the invention displays a viscosity superior to 1 Pa.s at very low shear rate (less than 0.1 s⁻¹). In one embodiment, the polyaphron of the invention displays a viscosity superior to 1 Pa.s at zero shear rate.

The polyaphron viscosity is measured using a rheometer equipment known by the skilled man of the art (e.g. Rotational rheometer Kinexus, Malvern UK), between 25 and 35°C, at atmospheric pressure (1 atm).

In one embodiment, the polyaphron exhibits a shear thinning behavior and the characteristics of a tixotropic material making it easier and more convenient to apply onto at least one eyelid of a subject.

### pH adjusting agent or buffer

In one embodiment, the polyaphron includes at least one pH adjusting agent or buffer selected from hydrochloric acid, citric acid, phosphoric acid, acetic acid, sodium hydroxide, potassium hydroxide, boric acid, borax, sodium carbonate, sodium hydrogen carbonate and the like. The amount of pH adjusting agent is function of the final pH value, comprised between 3.5 and 7.5. Also, the amount of pH adjusting agent is used in respect of the Pharmacopeia criteria and biological criteria.

### Preservative agent

In one embodiment, the polyaphron includes at least one preservative agent selected from benzalkonium chloride, benzyl alcohol, mercury salts, thiomersal, chlorhexidine, boric acid and/or a salt thereof or the like, as such or in combination. Also, the amount of preservative agent is used in respect of the Pharmacopeia criteria and biological criteria.

### Solubilizer

In one embodiment, the polyaphron includes at least one solubilizer selected from ethanol, polyethylene glycol, glycerol, propylene glycol, N-methyl pyrrolidone, glycofurol, dimethyl isosorbide. Also, the amount of solubilizer is used in respect of the Pharmacopeia criteria and biological criteria.

### Chelating agent

In one embodiment, the polyaphron includes at least one chelating agent selected from edetic acid and salts thereof, ethylene glycol tetraacetic acid and salts thereof, citric acid, metaphosphoric acid, pyrophosphoric acid, polyphosphoric acid, malic acid, tartaric acid, phytic acid, and salts thereof; more preferably at least one type selected from the group consisting of edetic acid, citric acid, metaphosphoric acid, polyphosphoric acid, and salts thereof; and particularly preferably a salt of edetic acid. Also, the amount of chelating agent is used in respect of the Pharmacopeia criteria and biological criteria.

### Water-in-oil polyaphron and manufacturing process

The polyaphron is a water-in-oil polyaphron wherein the continuous phase is a hydrophobic phase and the dispersed phase includes or consists of aphrons having a hydrophilic inner core. In this embodiment, the amount of the continuous phase may range from 2% to 50%, preferably 2% to 20% of hydrophobic phase, in weight by total weight of the polyaphron; the amount of aphrons may range from 50% to 98% w/w, preferably 70% to 98% w/w, even more preferably 80% to 98% w/w, in weight by total weight of polyaphron. In one embodiment, the water-in-oil polyaphron includes aphrons having mean diameter ranging from 0.1 to 100µm.

In one embodiment, the water-in-oil polyaphron of the invention is manufactured following the process of stirring the oily phase, and then adding dropwise the aqueous solution. Preferably, the aqueous solution is stirred at room temperature using magnetic stirring or propeller at 200 rpm to 1000 rpm, and the oily phase is added at a specific rate allowing the autocatalytic formation of aphrons to occur.

### Polyaphrons with active ingredient

The polyaphron comprises an active ingredient. In one embodiment, the active ingredient is a molecule of therapeutic interest.

In one embodiment, said active ingredient is selected from:
- **antiallergenics** such as sodium cromoglycate, antazoline, chlorpheniramine, cetirizine, olopatadine, epinastine, ketotifen, azelastine, emedastine, levocabastine, terfenadine, and loratadine;
- **anti-inflammatories** such as cortisone, hydrocortisone, hydrocortisone acetate, dexamethasone, dexamethasone 21-phosphate, dexamethasone palmitate, fluoroquinolone, prednisone, methylprednisolone, prednisolone acetate, fluorometholone, triamcinolone, betamethasone, loteprednol, flumethasone, beclomethasone, difluprednate and triamcinolone acetonide and their derivatives;
- **non-steroidal anti-inflammatories** such as salicylate, indomethacin, ibuprofen, diclofenac, flurbiprofen, oxicams, piroxicam and COX2 inhibitors such as rofecoxib, nimesulide, nepafenac;
- **beta adrenergic blockers** such as timolol and salts thereof including timolol maleate, levobunolol hydrochloride and betaxolol hydrochloride, betaxolol, atenolol, befunolol, metipranolol, forskolin, carteolol;
- **cytokines, interleukins, prostaglandins** (also antiprostaglandins, and prostaglandin precursors) such as latanoprost, bimatoprost, tafluprost or travoprost;
- **cyclosporines, sirolimus, tacrolimus;**
- **antioxidants** such as lutein, vitamins, especially vitamin A, coenzyme Q10, polyunsaturated fatty acids and derivatives thereof;
- **inhibitors of carbonic anhydrase** such as brinzolamide, dorzolamide, acetazolamide, methazolamide, dichlorphenamide;
- **antivirals,** such as idoxuridine, trifluorothymidine, acyclovir, valaciclovir, ganciclovir, cidofovir and interferon;
- **antibiotics** such as aminoglycosides, carbacephem, carbapenems, cephalosporins, glycopeptides, penicillins, polypeptides, quinolones, sulfonamides, tetracyclines, chlortetracycline, bacitracin, neomycin, polymyxin, gramicidin, cephalexin, oxytetracycline, chloramphenicol, kanamycin, rifampicin, tobramycin, gentamycin, ciprofloxacin, aminosides, erythromycin, ceftazidime, vancomycin, imipeneme; macrolides, azithromycin, clarithromycin, fluoroquinolones;
- **antibacterials** such as sulfonamides, sulfadiazine, sulfacetamide, sulfamethizole, sulfisoxazole, nitrofurazone and sodium propionate;
- and/or their derivatives; and/or their prodrugs; and/or their precursors; and/or acceptable salts thereof; alone or in combination.

In one embodiment, said active ingredient is selected from latanoprost, tafluprost, timolol, dorzolamide, olopatadine, epinastine, azithromycin, clarithromycin, cyclosporin A and sirolimus, dexamethasone, dexamethasone palmitate.

In one embodiment, said active ingredient is olopatadine. In one embodiment, said active ingredient is epinastine. In one embodiment, said active ingredient is clarithromycin. In one embodiment, said active ingredient is cyclosporin A. In one embodiment, said active ingredient is dexamethasone palmitate.

In one embodiment, the polyaphron stabilizes and/or preserves the active ingredient.

### Sterilization

In one embodiment, the polyaphron may be sterilized.

Non-limiting examples of sterilization methods are heating, such as by autoclaving, filtering or filtration, irradiation, and gas sterilization.

### Form

In one embodiment, the polyaphron of the invention may be a liquid, fluid, gel, powder, ointment, cream, patch, film formulation, or any delivery formulation suitable for palpebral administration.

Preferably, the polyaphron has a viscosity suitable for topical administration on eye lid skin, and is dispensed or administered to the subject in the form of a gel or a cream, or an ointment, or a patch, or in any form suitable for ophthalmic use by palpebral administration.

### Package

In one embodiment, the polyaphron is packaged in unitary doses; in another embodiment, the polyaphron is packaged in suitable multidose containers.

### Method

### Application onto the eyelid - Kit

In one aspect, this invention relates to a method for topically applying a polyaphron onto at least one eyelid, upper eyelid and/or lower eyelid, of a subject.

In one embodiment, the polyaphron is applied by spreading topically on the skin around the eye of the subject. In one embodiment, the polyaphron is applied by spreading on the eyelid of the subject. In one embodiment, the polyaphron is applied by spreading on the upper eyelid of the subject. In one embodiment, the polyaphron is applied by spreading on the lower eyelid of the subject.

In another embodiment, the polyaphron is applied with a spreading device, such as for example a brush or an applicator.

The present invention also relates to a kit comprising a container containing the polyaphron of the invention and the spreading device as described above.

### Transdermal delivery

In another aspect, this invention relates to a method for transdermal delivery of a polyaphron or an ingredient thereof onto the eye of a subject for treating an eye disease or an eye condition of said subject.

In one embodiment, the method is useful for delivering the polyaphron or an ingredient thereof to the surface of the eye or the anterior segment of the eye.

In one embodiment, the method is useful for eye care.

In one embodiment, the polyaphron comprises as additive a penetration enhancer, i.e. a compound which enhance the transdermal penetration of the polyaphron or of the ingredient thereof to the surface of the eye or the anterior segment of the eye.

### Sustained and/or controlled release of an ingredient

In another aspect, this invention relates to a method for sustained and/or controlled release of an ingredient onto the eye of a subject for treating an eye disease or an eye condition of said subject.

In one embodiment, the sustained and/or controlled release is obtained through application of polyaphrons of the invention containing the ingredient onto at least one eyelid, upper eyelid and/or lower eyelid, of a subject.

In one embodiment, the method is efficient to achieve sustained and/or controlled release administration of a therapeutic agent.

In one embodiment, the ingredient is released in a sustained and/or controlled manner for a period of time ranging from 1 hour to 2 weeks, preferably from 6 hours to 1 week, preferably from 12 hours to 5 days. In a specific embodiment, the ingredient is released in a sustained and/or controlled manner for a period of time ranging from 1 to 3 days.

In one embodiment, said sustained and/or controlled release kinetic may depend on the formulation of the polyaphron. In a specific embodiment, the release rate depends of the nature of the hydrophilic phase of the polyaphron. In a specific embodiment, the release rate depends of the nature of the hydrophobic phase of the polyaphron. In a specific embodiment, the release rate depends of the nature of a surfactant or mixture of surfactants comprised in the polyaphron. In a specific embodiment, the release rate depends of the concentration of a surfactant or mixture of surfactants comprised in the polyaphron.

In one embodiment, said sustained and/or controlled release kinetic may depend on the viscosity of the polyaphron. In a specific embodiment, the release rate decreases when the viscosity of the polyaphron increases.

In one embodiment, said sustained and/or controlled release kinetic may depend on the average globule size of the polyaphron. In a specific embodiment, the release rate decreases when the average globule size of the polyaphron decreases.

In an embodiment, said sustainable and/or controlled release kinetic may depend on the volume of polyaphron applied onto the eye of the subject.

In one embodiment, the sustainable and/or controlled release kinetic can be adapted to the exact need of the subject. In a specific embodiment, the release kinetic can be adapted to the exact need of the subject by selecting a surfactant or mixture of surfactants to be comprised in the polyaphron. In a specific embodiment, the release kinetic can be adapted to the exact need of the subject by selecting an appropriate concentration of surfactant or mixture of surfactants to be comprised in the polyaphron. In a specific embodiment, the release kinetic can be adapted to the exact need of the subject by changing the viscosity and/or the average globule size of the polyaphron.

In one embodiment, the method is useful for sustained and/or controlled release of the ingredient to the surface of the eye or the anterior segment of the eye.

In one embodiment, the method is useful for eye care.

### Eye diseases or eye conditions

In the meaning of the invention, eye diseases or eye conditions are dry eye condition such as for example dry-eye syndrome or chronic dry-eye diseases such as keratoconjunctivis sicca (KCS), atopic keratoconjunctivitis (AKC) and vernal keratoconjunctivitis (VKC), glaucoma, ocular inflammation conditions such as for example keratitis, corneal epithelium erosion, uveitis, including anterior uveitis, intraocular inflammation, allergy and dry-eye syndrome ocular infections, ocular infections, ocular allergies, corneal or conjunctival lesions, cancerous growth, diabetic macular edema, age-related macular degeneration, anesthesia of the cornea, mydriase of the pupil.

In one embodiment, the eye condition may be blepharitis, glaucoma, Meibomian glands disorders such as for example meibomian gland dysfunction (MGD) and dry eye condition such as for example dry-eye syndrome or chronic dry-eye diseases, diabetic keratopathy or neurotrophic keratopathy.

In one embodiment, the condition may be related to demodex folliculorum infection. In one embodiment, the condition is glaucoma. In one embodiment, the condition is anterior uveitis.

In another aspect, the polyaphrons of the invention are for use in the treatment of an eye disease or an eye condition.

In another aspect, the polyaphrons of the invention are for use in the manufacture of a medicine or medicament for the treatment of an eye disease or an eye condition.

In another aspect, the invention relates to a method of treatment of an eye disease or an eye condition wherein a therapeutically active amount of a therapeutic agent is administrated to a patient in need thereof through topical application of a polyaphron comprising the therapeutic agent. In one embodiment, the method comprises a step of topical administration of a composition comprising a polyaphron onto the upper and/lower or eyelid of the subject. In one embodiment, the topical application is palpebral application.

In one embodiment, the polyaphron is administrated once a day for 4 weeks.

In one embodiment, the palpebral administration through polyaphrons reduce the toxicity and/or side-effects of the treatment for the patient.

### DEFINITIONS

In the present invention, the following terms have the following meanings:
- **"Aphron"** refers to complex globule composed of an outer layer including surfactants encompassing an intermediate layer is composed of the same material as the continuous phase, itself encompassing a core made of the phase non-miscible with the continuous phase.
- **"Continuous phase"** refers to the phase encompassing the dispersed phase.
- **"Dispersed phase"** refers to the globule being dispersed in the continuous phase.
- **"Eyelid"** comprises upper eyelid, which starts from eyebrows to the lower limit identified by eyelashes basis, and lower eyelid which starts from infra-orbital region to the limit identified by eyelashes basis.
- **"MCT"** means Medium Chain Triglycerides.
- **"Molecule of therapeutic interest"** means any molecule having a particular interest for treating a pathology, a disease.
- **"ND"** means "not determined"
- **"Palpebral administration"** refers to the topical application onto the outer surface of at least one eyelid of a subject.
- **"Polyaphron"** refers to liquid globules called aphrons dispersed in a continuous phase.

### BRIEF DESCRIPTION OF THE DRAWINGS

**Figure 1** is a graph showing the results of *in vitro* efficacy test of clarithromycin loaded polyaphron **#28,** oily solution **#29** and ointment **#30** (Example 6).
**Figure 2** is a graph showing the results of *in vitro* penetration test of dexamethasone loaded polyaphrons **#31** to **#33** (Example 7).
**Figure 3** is a graph showing the results of *in vitro* skin penetration test of olopatadine HCl loaded polyaphrons **#21** to **#23** (Example 8).
**Figure 4** is a histogram showing the results the *in vivo* efficacy test of clarithromycin loaded polyaphron **#34** and solution **#39** (Example 10).
**Figure 5** is a histogram showing the results of *in vivo* efficacy test of clarithromycin loaded polyaphron **#34** and ointment **#40** (Example 11).
**Figure 6** is a histogram showing the results of *in vivo* efficacy test of clarithromycin loaded polyaphron **#34** and emulsion **#41** (Example 12).

### EXAMPLES

The present invention is further illustrated by the following examples

### Example 1: Oil-in-water polyaphrons without drug substance, not according to the invention

**Table 1: Composition of polyaphrons #1 to #6 (quantities are indicated in % weight /weight).**

| Composition # | **1** | **2** | **3** | **4** | **5** | **6** |
|---|---|---|---|---|---|---|
| **MCT** | 90 | 90 | 90 | 90 | 90 | 45 |
| **Triacetin** | | | | | | 45 |
| **Poloxamer 188** | 0.1 | 1.0 | | | | |
| **Poloxamer 407** | | | 0.1 | 1.0 | | |
| **Polyoxyl-40 stearate** | | | | | 1.0 | 0.1 |
| **Sorbitan oleate** | | | | | | 0.9 |
| **Water** | Qs 100 | Qs 100 | Qs 100 | Qs 100 | Qs 100 | Qs 100 |

### Example 2: Oil-in-water polyaphrons comprising cyclosporin A, not according to the invention

**Table 2: Composition of polyaphrons #7 to #15 (quantities are indicated in % weight /weight).**

| Composition # | 7 | 8 | 9 | 10 | 11 | 12 | 13 | 14 | 15 |
|---|---|---|---|---|---|---|---|---|---|
| **Cyclosporin A** | 1 | 1 | 1 | 1 | 1 | 1 | 1 | 1 | 1 |
| **MCT** | 88.1 | 88.1 | 88.1 | 88.1 | 88.1 | 89 | 88.1 | 77.2 | 88.1 |
| **Polyoxyethylene (4) lauryl ether** | 0.9 | | | | | | | | |
| **Sorbitan oleate** | | 0.9 | 0.9 | 0.9 | 0.9 | | 0.9 | 1.8 | 0.9 |
| **Poloxamer 188** | 0.1 | | | | | | | | |
| **Polyoxyl-40 stearate** | | 0.1 | | | | 1 | | | |
| **Sucrose laurate** | | | 0.1 | | | | | | |
| **Sucrose palmitate** | | | | 0.1 | | | | | |
| **Sucrose stearate** | | | | | 0.1 | | | | |
| **Alkyl polyglycoside** | | | | | | | 0.1 | | |
| **Polysorbate 80** | | | | | | | | 0.2 | |
| **Vitamine E TPGS** | | | | | | | | | 0.1 |
| **Water** | qs 100 | qs 100 | qs 100 | qs 100 | qs 100 | qs 100 | qs 100 | qs 100 | qs 100 |

### Example 3: Oil-in-water polyaphrons comprising dexamethasone palmitate, not according to the invention

**Table 3: Composition of polyaphrons #16 to #18 (quantities are indicated in % weight /weight).**

| Composition # | **16** | **17** | **18** |
|---|---|---|---|
| **Dexamethasone palmitate** | 0.8 | 0.8 | 0.8 |
| **MCT** | 88.3 | 89.2 | 89.2 |
| **Sorbitan oleate** | 0.9 | | |
| **Poloxamer 407** | | 1 | 0.1 |
| **Polyoxyl-40 stearate** | 0.1 | | |
| **Water** | qs 100 | qs 100 | qs 100 |

### Example 4: Water-in-oil polyaphrons including fluorescein sodium as hydrophilic marker or olopatadine HCl as drug substance, according to the invention

**Table 4: Composition of polyaphrons #19 and #23 (quantities are indicated in % weight. /weight).**

| Composition # | **19** | **20** | **21** | **22** | **23** |
|---|---|---|---|---|---|
| **Fluorescein sodium** | 0.0005 | 0.05 | | | |
| **Olopatadine HCl** | | | 0.5 | 0.5 | 0.5 |
| **Light mineral oil** | 19 | 19 | 17.9 | 17.9 | 17.9 |
| **Sorbitan oleate** | 1 | 1 | 2 | 2 | 2 |
| **Sucrose tristearate** | | | 0.1 | 0.1 | 0.1 |
| **Glycerol** | | | | 5.0 | |
| **PEG 400** | | | | | 5.0 |
| **Water** | qs 100 | qs 100 | Qs 100 | Qs 100 | Qs 100 |

### Example 5: Water-in-oil polyaphrons including epinastine as drug substance, according to the invention

**Table 5: Composition of polyaphrons #24 to #27 (quantities are indicated in % weight /weight).**

| Composition # | **24** | **25** | **26** | **27** |
|---|---|---|---|---|
| **Epinastine HCl** | 0.5 | 0.5 | 0.5 | 0.5 |
| **Light mineral oil** | 17.9 | 17.9 | 16.9 | 17.9 |
| **Sorbitan oleate** | 2 | 2 | 2 | 2 |
| **Sucrose tristearate** | 0.1 | 0.1 | 0.1 | 0.1 |
| **Isopropyl myristate** | | | 1 | |
| **Glycerol** | | | | 5 |
| **Diethylene glycol monoethyl ether** | | 2.5 | | |
| **PEG 400** | 5 | 2.5 | 5 | |
| **Water** | Qs 100 | Qs 100 | Qs 100 | Qs 100 |

The globule size distribution of each polyaphron was measured using a state of the art laser diffraction equipment (Helos Sympatec, Germany). The particle size distribution is determined on a volume basis (Dv). The viscosity of each polyaphron has been measured using a state of the art rheometer (Kinexus, Malvern UK).

Based on physical attributes of the compositions, displayed on Table 6, a correlation appears between the globule size distribution and the viscosity of the system: the globule size distribution is smaller when the composition is more viscous. These features may depend on either the qualitative or quantitative composition of the polyaphron.

**Table 6: Physical properties of polyaphrons #24 to 27.**

| Composition # | | **24** | **25** | **26** | **27** |
|---|---|---|---|---|---|
| **Globule size distribution (µm)** | | | | | |
| | **D(v,10) (µm)** | 1.8 | 2.9 | 2.5 | 1.8 |
| | **D(v,50) (µm)** | 7.2 | 16.7 | 12.3 | 7.7 |
| | **D(v,90) (µm)** | 37.9 | 48.8 | 38.3 | 33.6 |

| **Viscosity (Pa.s)** | | | | | |
|---|---|---|---|---|---|
| | **Initial viscosity η₀ (Pa.s)** | 4549 | 3398 | 5662 | 6744 |

### Example 6: In-vitro evaluation of the permeation of oil-in-water polyaphrons including clarithromycin (not according to the invention)

Transdermal permeation of clarithromycin was assessed using a Franz-cell equipment mounted with a Strat-M membrane purchased from Millipore (Molsheim, France). The Strat-M membrane is a synthetic membrane used for *in-vitro* testing simulating the skin permeation.

The diffusion of the clarithromycin from the polyaphron (Composition **#28**) was compared with the diffusion from oily solution (composition **#29**) and from an ointment (composition **#30).** Clarithromycin concentration in the receiver compartment over 72h was measured by Ultra Performance Liquid Chromatography (UPLC).

The following formulations were evaluated:

**Table 7: Composition of polyaphrons #28 to 30 (quantities are indicated in % weight /weight).**

| Composition # | **28** | **29** | **30** |
|---|---|---|---|
| **Clarithromycin** | 1 | 1 | 1 |
| **Capmul PG8** | 50 | 56 | |
| **MCT** | 39 | 43 | |
| **Light Mineral Oil** | | | 25 |
| **Heavy Mineral Oil** | | | 25 |
| **Petrolatum** | | | 49 |
| **CKC** | 0.005 | | |
| **Polysorbate 80** | 1 | | |
| **Water** | Qs 100 | | |

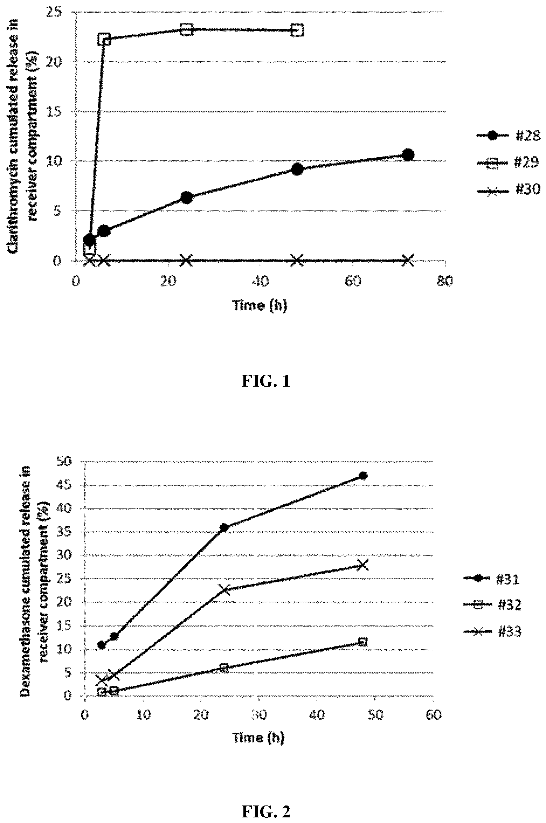

As disclosed on Figure 1, the application of ointment **#30** does not lead to transdermal permeation, while clarithromycin diffused from the polyaphron **#28.** Therefore, this experiment clearly demonstrates the superiority of polyaphrons over ointments in terms of skin penetration of a therapeutic agent.

Clarithromycin diffuses from the oily solution **#29** through the membrane, but such composition is not suitable for topical application on the eyelid, because its viscosity (25-33 mPa.s) is not high enough to allow the oily solution to remain to the eyelid. An oily solution will flow along the eyelid and/or into the eye. At the contrary, since the viscosity (> 1 Pa.s) of polyaphron **#28** is high enough to remain on the eyelid, it is thus suitable for palpebral administration.

In addition, as also shown in Figure 1, polyaphron **#28** is surprisingly able to efficiently release clarithromycin over more than 72h, whereas the oily solution **#29** immediately release the clarithromycin. This demonstrates that the use of polyaphrons can lead to a sustainable and controlled release of a therapeutic agent through the skin.

### Example 7: In-vitro evaluation of the permeation of oil-in-water polyaphrons including dexamethasone (not according to the invention)

Transdermal permeation of dexamethasone was assessed using a Franz-cell equipment mounted with a Strat-M membrane purchased from Millipore (Molsheim, France). The Strat-M membrane is a synthetic membrane used for *in-vitro* testing simulating the skin permeation.

The globule size distribution of each polyaphron was measured using a state of the art laser diffraction equipment (Helos Sympatec, Germany). The particle size distribution is determined on a volume basis (Dv). The viscosity of each polyaphron has been measured using a state of the art rheometer (Kinexus, Malvern UK).

The diffusion of the dexamethasone from polyaphrons having different compositions (Compositions **#31-33)** was compared. Dexamethasone concentration in the receiver compartment over 48h was measured by Ultra Performance Liquid Chromatography (UPLC).

The following formulations were evaluated:

**Table 8: Composition of polyaphrons #31 to 33(quantities are indicated in % weight /weight).**

| Composition # | **31** | **32** | **33** |
|---|---|---|---|
| **Dexamethasone** | 0.024 | 0.024 | 0.024 |
| **MCT** | 89 | 90 | 90 |
| **Sorbitan oleate** | 0.9 | | |
| **Poloxamer 407** | | 1 | 0.1 |
| **PEG-40 stearate** | 0.1 | | |
| **Water** | Qs 100 | Qs 100 | Qs 100 |

Based on physical attributes of the compositions, displayed on Table 9, a correlation appears between the globule size distribution and the viscosity of the system: the globule size distribution is smaller when the composition is more viscous. These features may depend on either the qualitative or quantitative composition of the polyaphron.

**Table 9: Physical properties of polyaphrons #31 to 33.**

| Composition # | | **31** | **32** | **33** |
|---|---|---|---|---|
| **Globule size distribution (um)** | | | | |
| | **D(v,10) (µm)** | 4.7 | 1.5 | 14.9 |
| | **D(v,50) (µm)** | 20.3 | 3.8 | 32.7 |
| | **D(v,90) (µm)** | 29.3 | 8.7 | 51.5 |

| **Viscosity (Pa.s)** | | | | |
|---|---|---|---|---|
| | **Initial viscosity η₀ (Pa.s)** | 22 | 300 | 5 |

Results disclosed on Figure 2 clearly demonstrate that polyaphrons are able to sustainably release dexamethasone through the skin over more than 48h. In addition, they demonstrate that the composition of the polyaphron impacts the release profile of the dexamethasone.

Surprisingly, the release kinetic depends of the nature of the surfactant. The release is faster with polyaphron **#31** where the surfactant is a mixture or PEG-40 stearate and sorbitan oleate, than with compositions **#32** and 33, where the surfactant is poloxamer.

Surprisingly, the release kinetics is also correlated with the viscosity of the composition and with the globule size distribution. Comparing compositions **#32** and **33,** increasing the relative concentration of surfactant decreases the globule size distribution and increase the viscosity. This leads to a slower kinetic with the more viscous polyaphron **#32,** having smaller particle size, than with the less viscous polyaphron #33, having higher particle size.

Therefore, the release kinetic from polyaphrons can be controlled by changing the qualitative and/or quantitative formulation of the polyaphron, e.g. by acting on the nature and/or the concentration of the surfactant.

### Example 8: In-vitro evaluation of the permeation of water-in-oil polyaphrons including olopatadine HCl

Transdermal permeation of olopatadine HCl was assessed using a Franz-cell equipment mounted with a Strat-M membrane purchased from Millipore (Molsheim, France). The Strat-M membrane is a synthetic membrane used for *in-vitro* testing simulating the skin permeation.

The diffusion of olopatadine HCl from polyaphrons having different compositions disclosed in Table 4 of Example 4 (Compositions **#21-23)** was compared. Olopatadine HCl concentration in the receiver compartment over 48h was measured by Ultra Performance Liquid Chromatography (UPLC).

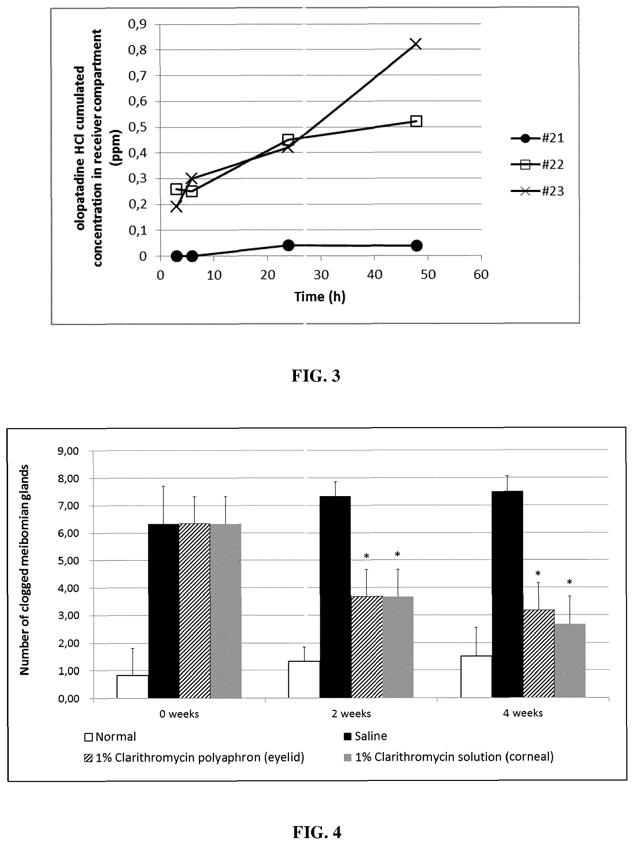

As showed in Figure 3, a sustained and controlled release is also obtained when diffusing olopatadine HCl through polyaphrons.

Surprisingly, addition of a hydrophilic co-solvent (e.g. glycerol, PEG 400) enhance the penetration through the *in vitro* Strat-M membrane, acting as a penetration enhancer.

### Example 9: Method of manufacturing of polyaphrons #34 to 38

Polyaphron **#34** to **38** incorporating clarithromycin were used for *in vivo* evaluation (Examples 10-13 below).

Polyaphron **#34** of Table 10 below was manufactured according to the following process: to an aqueous phase (8.995g) containing polysorbate 80SR (1g) stirred at 200 rpm was added dropwise the oily phase MCT (39g) containing CKC (0.005g), propylene glycol monocaprylate (50g) and clarithromycin (1g). The rate of addition of the oily phase at the start of the process was slow (approximately one drop every 7 seconds) but was speeded up once 20% of the oil phase had been added so that the total time to make the polyaphron was approximately 20 minutes.

Polyaphrons **#35** to **38** of Table 10 below were prepared according to the same manufacturing process.

**Table 10: Composition of polyaphron #34 to 38 (quantities are indicated in % weight /weight).**

| | **34** | **35** | **36** | **37** | **38** |
|---|---|---|---|---|---|
| **Clarithromycin** | 1 | 0.3 | 0.25 | 0.5 | - |
| **MCT** | 39 | 39 | 47.88 | 47.75 | 39 |
| **Propylene glycol monocaprylate** | 50 | 50 | 41.89 | 41.78 | 50 |
| **Polysorbate 80 SR** | 1 | 1 | 0.2 | 0.2 | 1 |
| **Water** | qs 100 | qs 100 | qs 100 | qs 100 | qs 100 |

Emulsions and ointment incorporating clarithromycin **#39** to **42** of Table 11 below have been prepared for comparison.

**Table 11: Composition of formulations #39 to 42 (quantities are indicated in % weight /weight).**

| | **Solution 39** | **Ointment 40** | **Emulsion 41** | **Emulsion 42** |
|---|---|---|---|---|
| **Clarithromycin** | 1 | 3 | 1 | 0.5 |
| **MCT** | 39 | - | | |
| **Propylene glycol monocaprylate** | 50 | - | 25 | 25 |
| **White petrolatum** | - | 77.5 | - | - |
| **Mineral oil** | - | 12.1 | - | - |
| **Polysorbate 80 SR** | | - | 2.5 | 2.5 |
| **PEG-40 stearate** | 0.1 | - | - | - |
| **Sorbitan monooleate** | - | - | 2.5 | 2.5 |
| **Sodium hydrogenophosphate** | 0.1 | | | |
| **Glycerol** | 2 | - | 1.2 | 1.2 |
| **Water** | qs 100 | - | qs 100 | qs 100 |

### Example 10: In vivo experiment - palpebral application of polyaphron #34 compared to dispensing solution #39 on cornea

Occlusion of meibomian glands is meibomian gland dysfunction (MGD) which is often responsible of dry eye, and may also contribute to blepharitis.

Polyaphron **#34** (1% of clarithromycin w/w) has been applied topically once a day for 4 weeks onto the eyelid of a subject presenting occluded meibomian glands. For comparison, solution **#39** (1% clarithromycin w/w) was dispensed on the cornea of the subject 3 times per day for 4 weeks. Each formulation was evaluated on 6 eyes (n=6). The results are shown on Figure 4.

Polyaphron **#34** (1% of clarithromycin w/w) reduced the number of occluded glands after four-week treatment in the same proportion as the solution **#39** (1% clarithromycin w/w) dispensed on the cornea did.

Moreover, it was clinically observed that the toxicity of polyaphron **#34** applied topically on the eyelid is lower than the toxicity of the solution **#39** directly dispensed on the cornea. Solution instilled onto the cornea triggered irritation and necrosis of corneal epithelium whereas no sign of corneal toxicity were reported with palpebral application of polyaphron **#34.**

### Example 11: In vivo experiment - palpebral application of polyaphron #34 compared to palpebral application of ointment #40

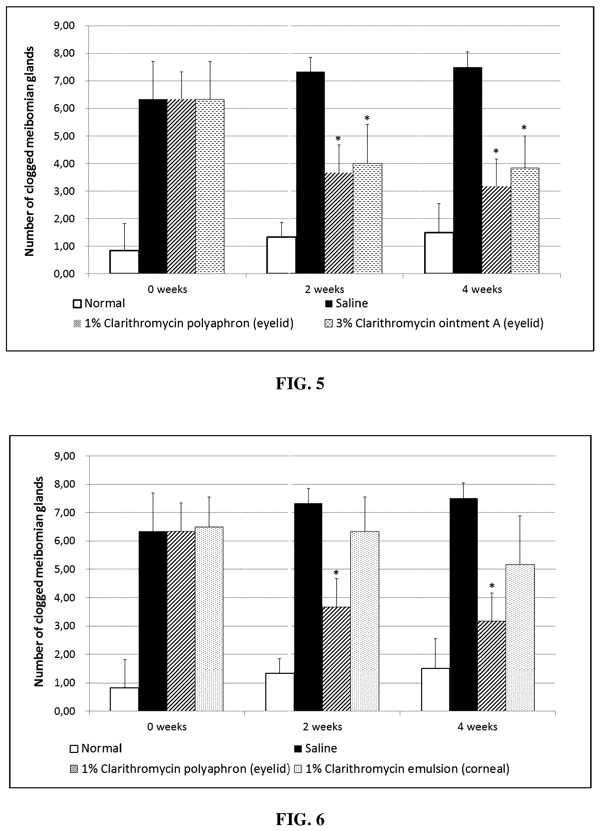

Polyaphron #34 (1% clarithromycin w/w) has been applied once per day for 4 weeks topically on the eyelid of a subject presenting occluded meibomian glands. In comparison, an ointment **#40** (3% clarithromycin w/w) has been applied topically on the eyelid of a subject once a day for 4 weeks. Each formulation was evaluated on 6 eyes (n=6). The results are shown on Figure 5.

Polyaphron **#34** reduced the number of occluded glands after four week treatment in the same proportion as the ointment **#40** does. However, the reduction in occluded cells number has to be balanced by the fact that the ointment **#40** is three times more concentrated than polyaphron **#34.** Consequently, when polyaphron **#34** is used, a reduced amount of clarithromycin in polyaphron is required to induce the same efficacy than the ointment **#40.**

Toxicity profile of polyaphron **#34** is similar to the toxicity profile of the ointment **#40,** even though said ointment is three times more concentrated in clarithromycin than said polyaphron. No corneal toxicity has been observed with polyaphron **#34.**

Therefore, palpebral application of polyaphron #34 allows the use of reduced amounts of clarithromycin and present a better safety profile.

### Example 12: In vivo experiment - palpebral application of polyaphron #34 compared to corneal application of emulsion #41

Polyaphron **#34** (1% clarithromycin w/w) has been applied topically once per day for 4 weeks on the eyelid of a subject presenting occluded meibomian glands. In comparison, emulsion **#41** (1% clarithromycin w/w) has been applied topically 3 times per day for 4 weeks on the eyelid of a subject. Each formulation was evaluated on 6 eyes (n=6). The results are shown on Figure 6.

Polyaphron **#34** reduced the number of occluded glands after a week treatment in a greater extent than emulsion **#41** does.

In addition, polyaphron **#34** exhibits an improved safety profile comparted to emulsion **#41** instilled on cornea. Indeed, no sign of corneal toxicity was reported following application of polyaphron **#34** onto eyelids whereas severe toxicity (i.e. corneal epithelium necrosis) was reported after instillation of emulsion **#41.**

### Example 13: Stability measurements by quantitative analysis of clarithromycin in polyaphron #37 and in an oil-in-water emulsion #42

Polyaphron **#37** (0.5% clarithromycin w/w) and oil-in-water emulsion **#42** (0.5% clarithromycin w/w) have been heated at 40°C and 60°C for a week. The quantity of clarithromycin has been measured at the end of the experiment and compared to the initial quantity. Results are indicated in percentage of the initial quantity of clarithromycin and are presented in Table 12.

**Table 12: Stability of polyaphron #37 and of emulsion #42 after one week experiment.**

| | Temperature (°C) | Dosage of clarithromycin (% vs T₀) |
|---|---|---|
| Polyaphron **#37** (0,5% clarithromycin w/w) | 40 | 98.8 |
| | 60 | 96.0 |
| Oil-in-Water Emulsion **#42** (0,5% clarithromycin w/w) | 40 | 90.2 |
| | 60 | 71.7 |

At 40°C, 99% of the active ingredient is recovered after a week experiment in polyaphron **#37,** whereas only 90% are recovered in the oil-in-water emulsion **#42.** At 60°C; only 72% of active ingredient is recovered in the oil-in-water emulsion **#42** whereas polyaphron **#37** still contains 96% of active ingredient.

Polyaphron #37 thus allows an enhanced stability of clarithromycin compared to the oil-in-water emulsion #42.

## Claims

1. Composition for use in the treatment of an eye disease or an eye condition of a subject by palpebral administration of said composition onto the upper and/or lower eyelid of said subject, said composition comprising a polyaphron;
wherein said polyaphron is a water-in-oil polyaphron comprising:
at least one hydrophilic phase;
at least one hydrophobic phase;
at least one non-ionic surfactant selected from alkyl polyglycol ethers; alkyl polyglycol esters; ethoxylated alcohols; polyoxyethylene sorbitan fatty acid esters; castor oil derivatives; polyoxyethylene fatty acid esters; polyoxyethylene glycol hydrogenated castor oils; polyoxyethylene glycol castor oils; sorbitan fatty acid esters; block copolymers of ethylene oxide and propylene oxide such as poloxamers, preferably poloxamer 188 or poloxamer 407; tyloxapol; polysorbates; sucrose alkyl esters; sucrose alkyl ether; short chain fatty acids mono- and diesters of glycerol; medium chain fatty acids; mono- and diesters of glycerol; long chain saturated fatty acids; mono- and diesters of glycerol; long chain unsaturated fatty acids mono- and diesters of glycerol; short chain fatty acids monoesters of propylene glycol; medium chain fatty acids monoesters of propylene glycol; long chain saturated fatty acids monoesters of propylene glycol; long chain unsaturated fatty acids monoesters of propylene glycol; polyoxylglycerides; polyoxyethylene alkyl esters; polyoxyethylene ethers; vitamin E polyethylene glycol succinate; alkylpolyglycosides; and mixtures thereof;
wherein the amount of non-ionic surfactant in the polyaphron ranges from 0.05 to 5% w/w in weight by total weight of the polyaphron;
at least one active ingredient; and
optionally at least one additive selected from antioxidants, osmotic, viscosity modulator, pH adjusting, buffering, preservative, solubilizing agents and chelating agents.

2. Composition for use according to claim **1,** wherein said at least one active ingredient is selected from latanoprost, timolol, dorzolamide, olopatadine, azithromycin, clarithromycin, cyclosporin A, sirolimus, dexamethasone and dexamethasone palmitate.

3. Composition for use according to claim **1** or claim **2,** wherein said use is an ophthalmic use.

4. Composition for use according to any one of claims **1** to **3,** wherein said composition is a liquid, fluid, gel, powder, ointment, cream, patch, or film formulation.

5. Composition for use according to any one of claims **1** to **4,** wherein said polyaphron includes globules having a mean size ranging from 0.1 to 100µm.

6. Composition for use according to any one of claims **1** to **5,** wherein said hydrophilic phase is an aqueous composition or water.

7. Composition for use according to any one of claims **1** to **6,** wherein said hydrophobic phase is selected from short chain fatty acids mono- di- and triesters of glycerol, medium chain fatty acids mono-, di- and triesters of glycerol, long chain saturated fatty acids mono-, di- and triesters of glycerol, long chain unsaturated fatty acids mono-, di- and triesters of glycerol, vegetable oils, almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, shark liver oil, squalane, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, fatty acid esters, short chain fatty acids mono- and diesters of propylene glycol, medium chain fatty acids mono- and diesters of propylene glycol, long chain saturated fatty acids mono- and diesters of propylene glycol, long chain unsaturated fatty acids mono- and diesters of propylene glycol, fatty alcohol, branched fatty alcohol, silicone oils, mineral oils, petrolatum, vitamin E, vitamin E acetate, tocopherol, tocopherol acetate, saturated fatty acids, unsaturated fatty acids, phospholipids, triacetin, and mixtures thereof.

8. Composition for use according to any one of claims **1** to **7,** wherein the hydrophobic phase comprises mineral oils; wherein said non-ionic surfactant comprises sorbitan oleate, sucrose tristearate, or a mixture thereof; wherein said active ingredient is olopatadine; and wherein said active ingredient is released in a controlled and/or sustained manner for a period of time ranging from 12 hours to 5 days.

9. Composition for use according to claim **8,** wherein said active ingredient is released in a controlled and/or sustained manner for a period of time ranging from 1 to 3 days.

10. Composition for use according to any one of claims **1** to **9,** wherein said composition is deposited, impregnated or coated on a spreading device for the application of said composition.

11. Composition for use according to claim **10,** wherein said spreading device for the application of said composition is a brush or an applicator.

12. Kit comprising a container containing a composition for use according to any one of claims **1** to **11,** and a spreading device for the application of said composition.

13. Kit according to claim **12,** wherein said spreading device for the application of said composition is a brush or an applicator.

## Patentansprüche

1. Zusammensetzung zur Verwendung bei der Behandlung einer Augenerkrankung oder eines Augenleidens eines Probanden durch palpebrale Verabreichung der Zusammensetzung auf das obere und/oder untere Augenlid des Probanden, wobei die Zusammensetzung ein Polyaphron umfasst;
wobei das Polyaphron ein Wasser-in-Öl-Polyaphron ist, umfassend:
mindestens eine hydrophile Phase;
mindestens eine hydrophobe Phase;
mindestens ein nichtionisches Tensid, ausgewählt aus Alkylpolyglykolethern; Alkylpolyglykolestern; ethoxylierten Alkoholen; Polyoxyethylensorbitanfettsäureestern; Rizinusölderivaten; Polyoxyethylenfettsäureestern; polyoxyethylenglykolhydrierten Rizinusölen; Polyoxyethylenglykol-Rizinusölen; Sorbitanfettsäureestern; Blockcopolymeren von Ethylenoxid und Propylenoxid wie Poloxameren, vorzugsweise Poloxamer 188 oder Poloxamer 407; Tyloxapol; Polysorbaten; Saccharosealkylestern; Saccharosealkylether; kurzkettigen Fettsäure-Mono- und Diestern von Glycerin; mittelkettigen Fettsäure-Mono- und Diestern von Glycerin; langkettigen gesättigten Fettsäure-Mono- und Diestern von Glycerin; langkettigen ungesättigten Fettsäure-Mono- und Diestern von Glycerin; kurzkettigen Fettsäure-Monoestern von Propylenglykol; mittelkettigen Fettsäure-Monoestern von Propylenglykol; langkettigen gesättigten Fettsäure-Monoestern von Propylenglykol; langkettigen ungesättigten Fettsäure-Monoestern von Propylenglykol; Polyoxylglyceriden; Polyoxyethylenalkylestern; Polyoxyethylenethern; Vitamin E-Polyethylenglykolsuccinat; Alkylpolyglycosiden; und Mischungen davon;
wobei die Menge des nichtionischen Tensids in dem Polyaphron von 0,05 bis 5 Gew.-% nach Gesamtgewicht des Polyaphrons beträgt;
mindestens einen Wirkstoff; und
optional mindestens ein Additiv, ausgewählt aus Antioxidantien, osmotischen Mitteln, Viskositätsmodulatoren, pH-Wert-Anpassungsmitteln, Puffern, Konservierungsmitteln, Lösungsvermittlern und Chelatbildnern.

2. Zusammensetzung zur Verwendung nach Anspruch **1,** wobei der mindestens einen Wirkstoff aus Latanoprost, Timolol, Dorzolamid, Olopatadin, Azithromycin, Clarithromycin, Cyclosporin A, Sirolimus, Dexamethason und Dexamethasonpalmitat ausgewählt ist.

3. Zusammensetzung zur Verwendung nach Anspruch **1** oder Anspruch **2,** wobei die Verwendung eine ophthalmologische Verwendung ist.

4. Zusammensetzung zur Verwendung nach einem der Ansprüche 1 bis 3, wobei die Zusammensetzung eine flüssige, fluide, gelartige, pulverförmige, salbenartige, cremige, pflasterartige oder filmartige Formulierung ist.

5. Zusammensetzung zur Verwendung nach einem der Ansprüche **1** bis **4,** wobei das Polyaphron Globuli mit einer mittleren Größe von 0,1 bis 100 µm enthält.

6. Zusammensetzung zur Verwendung nach einem der Ansprüche **1** bis **5,** wobei die hydrophile Phase eine wässrige Zusammensetzung oder Wasser ist.

7. Zusammensetzung zur Verwendung nach einem der Ansprüche **1** bis **6,** wobei die hydrophobe Phase ausgewählt ist aus kurzkettigen Fettsäure-Mono-, Di- und Triestern von Glycerin, mittelkettigen Fettsäure-Mono-, Di- und Triestern von Glycerin, langkettigen gesättigten Fettsäuren-Mono-, Di- und Triestern von Glycerin, langkettigen ungesättigten Fettsäure-Mono-, Di- und Triestern von Glycerin, Pflanzenölen, Mandelöl, Babassuöl, Johannisbeerkernöl, Borretschöl, Rapsöl, Rizinusöl, Kokosöl, Kabeljauleberöl, Maisöl, Baumwollsamenöl, Nelkenöl, Fischöl, Traubenkernöl, Senfkernöl, Haferöl, Olivenöl, Palmkernöl, Palmöl, Erdnussöl, Rapsöl, Safloröl, Sesamöl, Haileberöl, Squalan, Sojabohnenöl, Sonnenblumenöl, Walnussöl, Weizenkeimöl, hydriertem Rizinusöl, hydriertem Kokosöl, hydriertem Baumwollsamenöl, hydriertem Palmöl, hydriertem Sojaöl, teilweise hydriertem Sojaöl, hydriertem Pflanzenöl, Fettsäureestern, kurzkettigen Fettsäure-Mono- und Diestern von Propylenglykol, mittelkettigen Fettsäure-Mono- und Diestern von Propylenglykol, langkettigen gesättigten Fettsäure-Mono- und Diestern von Propylenglykol, langkettigen ungesättigten Fettsäure-Mono- und Diestern von Propylenglykol, Fettalkohol, verzweigtem Fettalkohol, Silikonölen Mineralölen, Vaseline, Vitamin E, Vitamin E-Acetat, Tocopherol, Tocopherolacetat, gesättigten Fettsäuren, ungesättigten Fettsäuren, Phospholipiden, Triacetin, und Mischungen davon.

8. Zusammensetzung zur Verwendung nach einem der Ansprüche **1** bis **7,** wobei die hydrophobe Phase Mineralöle umfasst; wobei das nichtionische Tensid Sorbitanoletat, Saccharose-Tristearat oder eine Mischung davon umfasst; wobei der Wirkstoff Olopatadin ist; und wobei der Wirkstoff in kontrollierter und/oder nachhaltiger Weise über einen Zeitraum von 12 Stunden bis 5 Tagen freigesetzt wird.

9. Zusammensetzung zur Verwendung nach Anspruch **8,** wobei der Wirkstoff in kontrollierter und/oder nachhaltiger Weise über einen Zeitraum von 1 bis 3 Tagen freigesetzt wird.

10. Zusammensetzung zur Verwendung nach einem der Ansprüche **1** bis **9,** wobei die Zusammensetzung auf eine Verteilervorrichtung zum Auftragen der Zusammensetzung aufgebracht, imprägniert oder beschichtet wird.

11. Zusammensetzung zur Verwendung nach Anspruch **10,** wobei die Verteilervorrichtung zum Auftragen der Zusammensetzung ein Pinsel oder ein Applikator ist.

12. Kit, umfassend einen Behälter, der eine Zusammensetzung zur Verwendung nach einem der Ansprüche **1** bis **11** enthält, und eine Verteilervorrichtung zum Auftragen der Zusammensetzung.

13. Kit nach Anspruch **12,** wobei die Verteilervorrichtung zum Auftragen der Zusammensetzung ein Pinsel oder ein Applikator ist.

## Revendications

1. Composition pour son utilisation dans le traitement d'une maladie oculaire ou d'une affection oculaire par administration palpébrale de ladite composition sur la paupière supérieure et/ou inférieure dudit sujet, ladite composition comprenant un polyaphron ;
dans laquelle ledit polyaphron est un polyaphron eau-dans-huile comprenant :
au moins une phase hydrophile ;
au moins une phase hydrophobe ;
au moins un tensioactif non-ionique choisi parmi les éthers d'alkylpolyglycol ; les esters d'alkylpolyglycol ; les alcools éthoxylés ; les esters d'acides gras de sorbitan polyoxyéthyléné ; les dérivés d'huile de ricin ; les esters d'acides gras de polyoxyéthylène ; les huiles de ricin hydrogénées de polyoxyéthylène glycol ; les huiles de ricin de polyoxyéthylène glycol ; les esters d'acides gras de sorbitan ; les copolymères séquencés d'oxyde d'éthylène et d'oxyde de propylène tels que les poloxamères, de préférence le poloxamère 188 ou le poloxamère 407 ; le tyloxapol ; les polysorbates ; les esters alkyliques de saccharose ; l'éther alkylique de saccharose ; les mono- et diesters d'acides gras à chaîne courte de glycérol ; les acides gras à chaîne moyenne ; les mono- et diesters de glycérol ; les acides gras saturés à chaîne longue ; les mono- et diesters de glycérol ; les mono- et diesters d'acides gras insaturés à chaîne longue de glycérol ; les monoesters d'acides gras à chaîne courte de propylène glycol ; monoesters d'acides gras à chaîne moyenne de propylène glycol ; monoesters d'acides gras saturés à chaîne longue de propylène glycol ; monoesters d'acides gras insaturés à chaîne longue de propylène glycol ; polyoxyglycérides ; esters alkyliques de polyoxyéthylène ; éthers de polyoxyéthylène ; succinate de polyéthylène glycol de vitamine E ; alkylpolyglycosides ; et leurs mélanges ;
dans lequel la quantité de tensioactif non-ionique dans le polyaphron varie de 0,05 à 5 % en poids par rapport au poids total du polyaphron ;
au moins un ingrédient actif ; et
optionnellement, au moins un additif choisi parmi les antioxydants, les agents osmotiques, les modulateurs de viscosité, les correcteurs de pH, les agents tampon, les conservateurs, les solubilisants et les agents chélateurs.

2. Composition pour son utilisation selon la revendication **1,** dans laquelle au moins un ingrédient actif est choisi parmi le latanoprost, le timolol, le dorzolamide, l'olopatadine, l'azithromycine, la clarithromycine, la cyclosporine A, le sirolimus, la dexaméthasone et le palmitate de dexaméthasone.

3. Composition pour son utilisation selon la revendication **1** ou la revendication **2,** dans lequel ladite utilisation est une utilisation ophtalmique.

4. Composition pour son utilisation selon l'une quelconque des revendications **1** à **3,** dans lequel ladite composition est une formulation liquide, fluide, gel, poudre, pommade, crème, patch ou film.

5. Composition pour son utilisation selon l'une quelconque des revendications **1** à **4,** dans lequel ledit polyaphron comprend des globules dont la taille moyenne est comprise entre 0,1 et 100 µm.

6. Composition pour son utilisation selon l'une quelconque des revendications **1** à **5,** dans lequel ladite phase hydrophile est une composition aqueuse ou de l'eau.

7. Composition pour son utilisation selon l'une quelconque des revendications **1** à **6,** dans lequel ladite phase hydrophobe est choisie parmi les mono-, di- et triesters d'acides gras à chaîne courte de glycérol, les mono-, di- et triesters d'acides gras à chaîne moyenne de glycérol, les mono-, di- et triesters d'acides gras saturés à chaîne longue de glycérol, les mono-, di- et triesters d'acides gras insaturés à chaîne longue de glycérol, les huiles végétales, l'huile d'amande, l'huile de babassu, l'huile de pépins de cassis, l'huile de bourrache, l'huile de canola, l'huile de ricin, l'huile de coco, l'huile de foie de morue, l'huile de maïs, l'huile de coton, l'huile d'onagre, l'huile de poisson, l'huile de pépins de raisin, l'huile de moutarde, l'huile d'avoine, l'huile d'olive, l'huile de palmiste, l'huile de palme, l'huile d'arachide, l'huile de colza, l'huile de carthame, l'huile de sésame, l'huile de foie de requin, le squalane, l'huile de soja, l'huile de tournesol, l'huile de noix, l'huile de germe de blé, l'huile de ricin hydrogénée, l'huile de coco hydrogénée, l'huile de coton hydrogénée, l'huile de palme hydrogénée, l'huile de soja hydrogénée, l'huile de soja partiellement hydrogénée, huile végétale hydrogénée, les esters d'acides gras, les mono- et diesters d'acides gras à chaîne courte de propylène glycol, les mono- et diesters d'acides gras à chaîne moyenne de propylène glycol, les mono- et diesters d'acides gras saturés à chaîne longue de propylène glycol, les mono- et diesters d'acides gras insaturés à chaîne longue de propylène glycol, les alcool gras, l'alcool gras ramifié, les huiles de silicone, les huiles minérales, la vaseline, la vitamine E, l'acétate de vitamine E, le tocophérol, l'acétate de tocophérol, les acides gras saturés, les acides gras insaturés, les phospholipides, la triacétine, et leurs mélanges.

8. Composition pour son utilisation selon l'une quelconque des revendications **1** à **7,** dans lequel la phase hydrophobe comprend des huiles minérales ; dans lequel ledit tensioactif non-ionique comprend de l'oléate de sorbitan, du tristéarate de saccharose, ou un mélange de ceux-ci ; dans lequel ledit ingrédient actif est l'olopatadine ; et dans lequel ledit ingrédient actif est libéré de manière contrôlée et/ou prolongée pendant une durée allant de 12 heures à 5 jours.

9. Composition pour son utilisation selon la revendication **8,** dans lequel ledit ingrédient actif est libéré de manière contrôlée et/ou prolongée pendant une période allant de 1 à 3 jours.

10. Composition pour son utilisation selon l'une quelconque des revendications **1** à **9,** dans lequel ladite composition est déposée, imprégnée ou revêtue sur un dispositif d'application de ladite composition.

11. Composition pour son utilisation selon la revendication **10,** dans lequel ledit dispositif d'application de ladite composition est un pinceau ou un applicateur.

12. Kit comprenant un récipient contenant une composition pour son utilisation selon l'une quelconque des revendications **1** à **11,** et un dispositif d'application de ladite composition.

13. Kit selon la revendication **12,** dans lequel ledit dispositif d'application de ladite composition est un pinceau ou un applicateur.
